# EUROPÄISCHE PATENTANMELDUNG

(11) **EP 3 517 601 A1**
(43) Veröffentlichungstag der Anmeldung: **31.07.2019**
(21) Anmeldenummer: 18153844.8
(22) Anmeldetag: 29.01.2018
(51) Int. Cl.: C12M 1/00, C12M 1/12, C12M 1/02, F28F 21/06

(54) **VERFAHREN UND ANLAGE ZUR HERSTELLUNG VON MIKROALGEN**

(71) Anmelder: Bioprodukte Prof. Steinberg GmbH, 06217 Merseburg (DE)
(72) Erfinder: Steinberg, Karl-Hermann, 06217 Merseburg (DE)
(74) Vertreter: Patentanwälte Bressel und Partner mbB

(57) **Zusammenfassung**

Die Erfindung betrifft ein Verfahren und eine Anlage zur Herstellung von Mikroalgen in einem tubulären Dünnschichtphotobioreaktor mit aktiver Temperaturführung und Kreislaufführung einer Mikroalgen-Kultivationslösung in phototropher und mixotropher Kultivationsweise, wobei als Dünnschichtphotobioreaktor parallel angeordnetee Glasröhren eingesetzt werden, in denen ein flexibler Schlauch verlegt ist, der frei in der Mikroalgen-Kultivationslösung schwimmt und durch den ein Temperiermedium gepumpt wird, welches periodisch durch Luft ersetzt wird und die Kultivationslösung durch einen Kreislaufbehälter geführt wird, in dem ein spiralförmiges Temperierelement angeordnet ist.

## Beschreibung

Die Erfindung betrifft ein Verfahren und eine Anlage zur Herstellung von Mikroalgen in einem tubulären Dünnschichtphotobioreaktor mit aktiver Temperaturführung und Kreislaufführung einer Mikroalgen-Kultivationslösung in phototropher und mixotropher Kultivationsweise, nach den Oberbegriffen der Ansprüche 1 und 12.

Mikroalgen sind wertvolle Nahrungs- und Futtermittel, die zukünftig einen großen Beitrag zur Versorgung der wachsenden Erdbevölkerung leisten werden. Mikroalgen wachsen im Wasser. Ihre Herstellung benötigt wesentlich weniger Fläche als die Herstellung vergleichbarer landwirtschaftlicher Produkte.

Zur industriellen Produktion von Mikroalgen sind bereits mehrere Verfahren bekannt und angewendet.

So sind Verfahren zur Herstellung von Mikroalgen in natürlichen und künstlichen Teichen und Becken bekannt (H. Matsumoto et al., Applied Biochemistry and Biotechnology, Vol. 51/52, 681, 1995). Diese haben den Vorteil geringer Investitionskosten und niedriger Betriebskosten, aber die Nachteile von unvermeidbaren Kontaminationen durch Lebewesen, Staubeinwehungen, von Aufsalzung durch Verdampfungsverluste, von geringer Produktivität von ca. 10 g Biomasse pro m²d und von niedriger Produktqualität.

Eine Verbesserung der Produktqualität ist allerdings erreichbar, wenn die offenen Becken in einem Gewächshaus betrieben werden (DE 10 2014 011 317 A1). Dann steigen allerdings die Investitionskosten, die Kontaminationsgefahr ist geringer, aber die anderen Nachteile bleiben bestehen.

Verfahren zur Herstellung von Mikroalgen in geschlossenen Systemen sind seit einigen Jahrzehnten bekannt (u.a. DE 198 14 253 C2). Sie haben den prinzipiellen Vorteil der Vermeidbarkeit von Kontaminationen, der verfahrenstechnischen Optimierbarkeit und Kontrolle, der höheren Produktivität und der besseren Produktqualität. Nachteile sind höhere Investitionskosten und auch höhere Betriebskosten.

Ein bewährtes Verfahren ist die Kultivation von Mikroalgen im geschlossenen System von röhrenförmigen Dünnschichtgefäßen als Photobioreaktoren gemäß EP 1 040 182 B1. Es ermöglicht die Herstellung verschiedener Mikroalgenspezies in hoher Reinheit bei guter und stabiler Produktivität von täglich 0,2 bis 0,7 g Mikroalgenbiomasse pro I Kultivationslösung. Dieses Verfahren ist geeignet für Standorte in gemäßigten Klimazonen. Es ist wenig geeignet für Zonen mit starken Temperaturspitzen im Jahresverlauf und auch im Tagesverlauf, da die Mikroalgen je nach Spezies optimale Wachstums- und Lebenstemperaturen haben. Einige der für die Ernährung wichtigen Spezies sterben bei Temperaturen oberhalb von 38 °C auch in einer Kultivationslösung ab. Die Anordnung der Photobioreaktoren in einem Gewächshaus ist für die praktische Durchführung Bedingung.

Einen Fortschritt dazu stellt der in der Anmeldung DE 10 2009 045 851 A1 beschriebene Schlauch-Photobioreaktor zur Kultivierung von phototrophen Makro- und Mikroorganismen dar. Der wesentliche Vorteil besteht in der Verwendung von transparenten oder transluzenten Schläuchen als röhrenförmige Dünnschichtgefäße für Photobioreaktoren, vorzugsweise von flexiblen Silikongummi-Doppelkammerschläuchen. Diese Schläuche können mit geringem technischem Aufwand so zu einem Photobioreaktor-Modul angeordnet werden, dass sie entsprechend der geographischen Lage des Standorts, den gegebenen Klimabedingungen wie Sonnengang und Temperatur angepasst werden können. Durch die Verwendung der Doppelkammerschläuche kann eine Temperierung des eigentlichen Dünnschichtgefäßes für die Photosynthese erreicht werden. Wesentliche Nachteile der Verwendung von Doppelkammerschläuchen bestehen in der technischen Anbindung der Kammern an die Zu- und Abführung des Temperiermediums und des Kultivationsmediums, ohne dass eine unerwünschte Vermischung beider Medien stattfindet. Dazu werden in der Anmeldung DE 10 2009 045 851 A1 keine Vorschläge gemacht. Der Nachteil der technisch aufwendigen Anbindung des inneren und des äußeren Schlauchs wird ebenfalls nicht gelöst.
Die in dieser Anmeldung ebenfalls beanspruchte Verwendung von einem Innenschlauch, der durch Stege im äußeren Schlauch geführt wird, hat ebenfalls erhebliche Nachteile, weil durch diese Maßnahme ein Strömungswiderstand eintritt und die Ablagerung der Mikroalgen und das damit verbundene "Fouling" begünstigt wird. Fouling verursacht erhebliche Kosten. Diese Kosten entstehen zunächst durch verschlechterten Licht- und Wärmedurchgang. Weiterhin sind aber auch die ökologischen Kosten auf Grund des notwendigen Einsatzes von Bioziden zur Verhinderung des Biofouling oder des erhöhten Energieeinsatzes zur Kompensation der durch Fouling verursachten Minderleistung zu sehen.

Bekannt ist auch ein Verfahren, in dem zunächst ein Inokulat in einem geschlossenem Photobioreaktor angezüchtet wird und anschließend in einem Fermenter ohne Belichtung unter Zusatz von Glucose Wachstum bis zu einer hohen Mikroalgenkonzentration erreicht wird (WO 2009/149794 A1)

Ähnlich beschreibt DE 10 2008 059 562 A1 ein Verfahren zur Herstellung von Mikroalgen, bei dem anfangs eine Stammkultur durch Multiplikation in Photobioreaktoren unter künstlicher Belichtung hergestellt wird, anschließend in einem Fermenter ohne Belichtung unter Zusatz von Glucose heterotroph vermehrt und schließlich in einem großen Photobioreaktor unter Nutzung von Sonnenlicht mixotroph bis zu einer erntereifen Mikroalgenkonzentration fertig kultiviert wird.

Als Photobioreaktoren werden nicht nur tubuläre Dünnschichtreaktoren vorgeschlagen, sondern auch Plattenreaktoren (DE 200 17 229 U1), z.B. aus Acrylglas (WO 2015/032389 A1). Durch Einbau von Schikanen in den Kreislauf der Kultivationslösung soll die Rückvermischung vermindert werden. Zur Erhöhung der Produktivität wird zusätzlich zum Tageslicht die künstliche Beleuchtung mit LED-Kunstlicht bestimmter Wellenlängen vorgeschlagen. Eine Temperierung der Reaktoren wird nicht beschrieben. Plattenreaktoren besitzen den prinzipiellen Nachteil, dass das für die Photosynthese unbedingt erforderliche Licht nur von zwei Seiten eindringen kann, was die Syntheseleistung nicht optimal ausschöpft.

Nachteile der Verwendung von Verfahren mit Fermentern sind die hohen Investitionskosten, die kosten für das Lebensmittel Glucose und die Ermüdung der Stammkulturen mit zunehmender Zahl der Kultivationszyklen. Es muss nach einigen Zyklen stets eine frische, in externen Photobioreaktoren gezüchtete Stammkultur zur Inokulation verwendet werden, weil sonst die für Mikroalgen typischen Wirkstoffe, wie z.B. Chlorophyll, nicht mehr in ausreichender Menge gebildet werden können.

Die Nachteile der meisten bekannten Verfahren bestehen auch darin, dass sie offenbar nur für die Herstellung kleiner Mengen in kleinen Anlagen (Labor- bzw. Pilotanlagen) geeignet sind. Maßnahmen, die für die Herstellung von Mikroalgen im großtechnischen Maßstab, also für die industrielle Produktion geeignet scheinen, werden im Stand der Technik nicht oder unzureichend beschrieben. Insbesondere werden die erforderlichen Investitionskosten und der erforderliche Energieaufwand nicht so beschrieben, dass auch hohe Mikroalgenmengen bei hoher Qualität unter Berücksichtigung der Wirtschaftlichkeit erreicht werden können.

Weitere Nachteile einiger beschriebener Verfahren zur Herstellung von Mikroalgen sind die vorgeschlagenen Materialien für die Photobioreaktoren. Insbesondere wird hierzu auf den in der Druckschrift DE 10 2009 045 851 A1 beschriebenen Schlauch-Photobioreaktor verwiesen. Synthetische Polymere wie Polymethacrylat, Polyäthylen, Silikongummi, Polystyrol und weitere haben im Vergleich zu Glas den prinzipiellen Nachteil für Photobioreaktoren zur Herstellung von Mikroalgen für die Ernährung, dass sie keine Spuren von Monomeren, aus denen sie hergestellt sind, enthalten dürfen. Diese könnten nach und nach durch Auslaugung (Leaching) in das Produkt Mikroalgen abgegeben werden. Außerdem werden einige dieser Polymermaterialien unter Verwendung von Weichmachern hergestellt. Auch diese können an das Produkt langsam abgegeben werden. Eine restlose Entfernung von Monomeren bzw. Weichmachern ist zwar möglich, aber aufwendig und verteuert das Material für die Photobioreaktoren erheblich.

Polymere sind außerdem empfindlich gegen den Anteil ultravioletter Strahlung im Sonnenlicht und altern leichter als z.B. Glas. Glas ist zudem billiger als viele der vorgeschlagenen Polymere für Photobioreaktoren. Glas hat sich als technische Lösung für Photobioreaktoren zur Herstellung von Mikroalgen seit Jahrzehnten in der Praxis bewährt (www.algomed.de), Auslaugung und Alterung wurden nicht beobachtet. Ein Nachteil von Photobioreaktoren aus Glas ist jedoch, dass die Glasrohre nicht beliebig in kostengünstige geometrische Formen für Photobioreaktoren gebracht werden können.

Ein weiterer Nachteil von Photobioreaktoren aus Glasrohren für die Herstellung von Mikroalgen ist, dass bisher keine technisch vernünftige und kostengünstige direkte Temperierung bekannt ist. Ummantelungen durch ein äußeres Glasrohr nach dem Prinzip des Liebigkühlers (DE 10 2007 012 745 A1) sind zwar bekannt, aber teuer und für industrielle Anlagen technisch zu aufwendig.

Wegen der Notwendigkeit von Sonnenlicht für die Photosynthese in industriellen Photobioreaktoren ist eine übliche technische Lösung die Anordnung der Photobioreaktoren in Gewächshäusern und deren Temperierung. Das ist eine kosten- und energieintensive Lösung, die außerdem nicht für jeden Standort vernünftig ist.

Die Aufgabe der Erfindung besteht deshalb darin, die oben beschriebenen Nachteile der bekannten Verfahren und Anlagen zu überwinden und ein Verfahren sowie eine Anlage vorzustellen, die für die industrielle biologische, insbesondere für photochemische Anwendung geeignet sind.

Erfindungsgemäß werden die Nachteile der bekannten Lösungen durch die Merkmale der Ansprüche 1 und 12 überwunden.

Demnach besteht die Erfindung in einem Verfahren zur Herstellung von Mikroalgen in einem tubulären Dünnschichtphotobioreaktor, der aus parallel angeordnete Glasröhren besteht, wobei die Mikroalgen ausgehend von einem Misch- und Ausgangsbehälter im Kreislauf geführt werden und dabei in einer Kultivationslösung kultiviert werden, wobei die Kultivation der Mikroalgen in dem tubulären Dünnschichtphotobioreaktor bei aktiver Temperaturführung und Kreislaufführung durch eine Innenkühlung der Kultivationslösung in phototropher oder mixotropher Fahrweise erfolgt. Zur aktiven Temperaturführung in den Glasröhren wird dazu ein flexibler Schlauch verlegt, der in der Kultivationslösung schwimmt und durch den ein Temperiermedium gepumpt wird. Vorteilhaft wird durch das Temperiermedium die Kultivationstemperatur oberhalb der minimalen Wachstumstemperatur und unterhalb der maximalen Wachstumstemperatur des jeweilig kultivierten Mikroalgentyps gehalten.
Nach einem besonderen Merkmal der Erfindung wird das Temperiermedium in dem flexiblen Schlauch periodisch durch Luft ersetzt wird. Die Luftimpulse führen zur periodischen Anhebung des flexiblen Schlauchs und verhindern dabei vorteilhaft das Fouling, weil die Absetzung eines Biofilms verhindert wird.

Der Nachteil des offenbar bisher nicht gelösten hohen technischen Aufwands der Durchführung eines inneren Schlauchs oder einer der beiden Kammern eines Doppelkammerschlauchs bei den bereits bekannten tubulären Dünnschichtphotobioreaktoren in und aus dem Reaktor wird dadurch gelöst, dass der flexible Innenschlauch einzeln oder als Bünde am oberen oder unteren Ende kammerförmiger An- und Abströmer der parallel angeordneten Röhren mittels klassischer Installationstechnik durch die Kultivationslösung hindurch nach außen geführt wird, sodass eine Vermischung von Temperiermedium oder Luft mit der Kultivationslösung unmöglich ist.
Dabei ist vorgesehen, dass in den Misch- und Ausgangsbehälter im Kreislauf der Kultivationslösung zur aktiven Temperaturführung ein spiralförmiges Temperierelement zum Kühlen oder Heizen angeordnet wird, wobei als spiralförmiges Temperierelement ein aus einem inerten Metallrohr bestehendes Temperierelement eingesetzt wird, welches von einem Temperiermedium durchflossen wird. Der flexible Schlauch und das spiralförmige Temperierelement können dabei von dem gleichen Temperiermedium durchflossen werden. Das Gesamtvolumen des durch den flexiblen Schlauch und das spiralförmige Temperierelement strömenden Temperiermediums beträgt dabei zwischen 1 % und 20 %, vorzugsweise 5 % des Kultivationslösungsvolumens. Durch den flexiblen Schlauch kann aber auch ein anderes Temperiermedium als durch das spiralförmige Temperierelement im Kreislauf der Kultivationslösung geleitet werden.

Nach einem besonderen Merkmale ist vorgesehen, dass der flexible Schlauch und/oder das spiralförmige Temperierelement auf ihren äußeren Oberflächen ganz oder teilweise mit einem Schutz gegen die Ablage eines Biofilms versehen werden.

Die Nachteile der bisher bekannten Verfahren, dass bei durch jahreszeitlich oder tageszeitlich bedingten Temperaturspitzen, die niedriger oder höher liegen als den optimalen Wachstumstemperaturen von Mikroalgen entspricht, kultiviert wird, werden durch die erfindungsgemäße aktive Temperaturführung (Innenkühlung) mittels in dem tubulären Dünnschichtphotobioreaktor angeordneten, von einem Temperiermedium durchströmten, flexiblen Schlauch, der in der Kultivationslösung schwimmt, überwunden.

Dadurch kann sowohl ein Absterben der Mikroalgen durch zu hohe Temperaturen vermieden werden als auch in gemäßigten Breiten früher im Jahr und auch länger im Jahr eien effektive Kultivierung von Mikroalgen erreicht werden. Beide Vorteile der aktiven Temperaturführung erhöhen sowohl die Produktqualität als auch die Produktivität im Vergleich zu bekannten Verfahren.

Durch die erfindungsgemäße Lösung der aktiven Temperaturführung können künftig Mikroalgen in industriellem Maßstab kostengünstig auch unter klimatischen Bedingungen kultiviert werden, unter denen es bisher nicht wirtschaftlich möglich war.

Die Nachteile von Plattenreaktoren werden durch die an sich schon bekannte Verwendung von tubulären Dünnschichtphotobioreaktoren aus Glasröhren überwunden. Durch die Verwendung von Röhren aus Glas wird auch der prinzipielle Nachteil der Verwendung von Röhren oder Schläuchen aus organischen Polymeren überwunden. Der Nachteil des hohen Preises der bisher vorgeschlagenen Verwendung von hochwertigen, aber mehrfach teureren Doppelschlauchreaktoren aus Silikongummi wird durch die Verwendung von Glasröhren mit einem innen angeordneten flexiblen von einem Temperiermedium durchströmten schwimmenden Schlauch überwunden.

Der potentiellen Ablagerung von Mikroaltenbiomasse in den tubulären Dünnschichtphotobioreaktoren aus Glasröhren im erfindungsgemäßen Verfahren im Dauerbetrieb wird sowohl durch die Wahl von hochwertigem Polymermaterial als Material des flexiblen Schlauchs als auch durch einen besonderen Schutz auf den äußeren Oberflächen sowie durch den kurzzeitigen Ersatz des Temperiermediums durch Luft begegnet, weil durch die Materialwahl eine Ablagerung vermindert wird und der Schlauch innerhalb der Glasröhren periodisch durch Auftrieb und Pulsation bewegt wird. Das "Fouling" wird dadurch verhindert bzw. zumindest verringert.
Die Merkmale der erfindungsgemäßen Anlage ergeben sich bereits aus den oben beschriebenen Verfahrensmerkmalen sowie den auf die Anlage bezogenen Ansprüchen und den Ausführungsbeispielen.

Die Erfindung wird in den nachfolgenden Ausführungsbeispielen näher beschrieben.

### Beispiel 1

In einem tubulären Dünnschichtphotobioreaktor werden Mikroalgen der Spezies Chlorella vulgaris nach dem erfindungsgemäßen Verfahren kultiviert.

Der tubuläre Dünschichtphotobioreaktor besteht im photoaktiven Teil aus Röhren aus auslaugungsbeständigem Borosilikatglas mit einem Außendurchmesser von 60 mm und einem Innendurchmesser von 54 mm. Die Glasröhren sind U-förmig ausgebildet, jeder Schenkel ist 50 m lang. 22 dieser U-förmigen Glasröhren sind in einem Abstand von jeweils 20 cm in einem Gestell übereinander angeordnet. Der Zustrom der Mikroalgenkultivationslösung in die und der Abstrom aus den 22 Glasröhren erfolgt jeweils durch eine kammförmige Sammelleitung. In allen Glasröhren ist ein flexibler Schlauch aus lebensmittelechtem Polyethylen mit einem Außendurchmesser von 20 mm und einem Innendurchmesser von 16 mm für die Aufnahme eines Temperiermediums beweglich angeordnet. Der flexible Schlauch in den Glasröhren wird jeweils einzeln bis in die Kammförmigen Sammelleitungen geführt und am Kopf bzw. am Boden der Sammelleitungen der Kultivationslösung mittels klassischer Installationstechnik durch die Verschlussdeckel herausgeführt, sodass schließlich jeweils 22 Schläuche außerhalb der Kultivationslösung das Gestell mit den 22 Glasröhren verlassen und zu einem einzigen Versorgungssystem für das Temperiermedium zusammengeführt werden.

Das Temperiermedium ist vorzugsweise Wasser, das in einer üblichen, externen Kühl- bzw. Heizvorrichtung auf die erforderliche Vorlauftemperatur gebracht wird. In den Vorlauf ist ein Ventilsystem angeordnet, das gesteuerte Luftpulse in das Temperiermedium einzuspeisen ermöglicht.

Eine Einheit des tubulären Dünnschichtphotobioreaktors, bestehend aus 12 Gestellen mit jeweils 22 der U-förmigen Glasrohrsystemen wie oben beschrieben, mit den kammerförmigen An- und Abstromsystemen, den Sammelleitungsverbindungen, dem Versorgungssystem für das Temperiermedium und einem Sammelbehälter im Kreislauf der Kultivationslösung ist in einem Gewächshaus aufgestellt.

Die Kultivationslösung wird im Kreislauf durch den Dünnschichtphotobioreaktor gepumpt. Im Sammelbehälter ist eine spiralförmige Temperiereinheit aus Edelstahlrohr mit 60 mm Außendurchmesser und mit einer Wandstärke von 2 mm, bestehend aus 30 Windungen mit jeweils 2 m Durchmesser angeordnet. Die spiralförmige Temperiereinheit kann wahlweise von dem gleichen Temperiermedium durchströmt werden wie der flexible Schlauch in den Glasröhren.

Im Frühjahr, auf einem Standort 52° nördlicher Breite mit einer durchschnittlichen Sonnenscheindauer von 5,2 h/d, wurde die Kultivation von Chlorella-Mikroalgen nach dem erfindungsgemäßen Verfahren durchgeführt. Zu Beginn der Kultivation einer Stammkultur mit 0,3 g/l (trockener Biomasse) betrug die Außentemperatur 12 °C und die Temperatur im Gewächshaus 15 °C. Das gesamte System zur aktiven Temperaturführung wurde eingeschaltet, die Vorlauftemperatur des Temperiermediums betrug 32 °C. Nach 3 h hatte die Kultivationslösung eine Temperatur von 26 °C erreicht. Diese Temperatur wurde durch automatische Regelung des Temperiermediums aufrechterhalten. Alle 45 Minuten wurde für jeweils 5 Minuten Luft anstelle des Temperaturmediums durch die flexiblen Schläuche geleitet. Nach 3 Tagen betrug die Mikroalgendichte 3,0 g/l. Die Zuwachsrate betrug 0,9 g/ld. Derartig hohe Zuwachsraten sind für solche Mikroalgenkulturen zu diesen Jahreszeiten bisher nicht beschrieben. Durch Ernte wurde diese Kultivation beendet.

### Beispiel 2

Das gleiche, in Beispiel 1 beschriebene Dünnschichtphotobioreaktorsystem, aber ohne das System zur aktiven Temperaturführung, wurde am gleichen Standort im Frühjahr für die Kultivation von Chlorella-Mikroalgen betrieben. Zu Beginn der Kultivation einer Stammkultur mit 0,4 g/l (trockener Biomasse) betrug die Temperatur im Gewächshaus 16 °C. Während der Kultivationsdauer von 3 Tagen stieg die Temperatur im Gewächshaus auf maximal 22 °C. Am Ende der 3-tägigen Kultivation betrug die Biomassekonzentration 1,4 g/l. Die Zuwachsrate betrug 0,33 g/Id.

### Beispiel 3

Das gleiche, in Beispiel 1 beschriebene Dünnschichtphotobioreaktorsystem wurde am gleichen Standort im Hochsommer mit der gleichen Stammkultur von Chlorella-Mikroalgen und einer Startkonzentration von 0,2 g/l erfindungsgemäß betrieben.

Die Starttemperatur der Kultivationslösung betrug 27 °C, die Außentemperatur mittags 36 °C, im Gewächshaus 42 °C. Nach Einschalten des gesamten Systems zur aktiven Temperaturführung mit einer Vorlauftemperatur von 12 °C wurde die Temperatur der Kultivationslösung auf 32 °C begrenzt und 3 Tage so gehalten. Alle 45 Minuten wurde für jeweils 5 Minuten Luft anstelle des Temperiermediums durch die flexiblen Schläuche geleitet. Nach 3 Tagen betrug die Mikroalgendichte 3,2 g/l. Die Zuwachsrate betrug 1 g/Id. Die mikroskopische Untersuchung der Kultivationslösung ergab 99,6 % intakter Chlorella-Zellen. Durch Ernte wurde diese Kultivation beendet.

### Beispiel 4

Das gleiche, in Beispiel 1 beschriebene Dünnschichtphotobioreaktorsystem, aber ohne das System zur aktiven Temperaturführung, wurde am gleichen Standort im Hochsommer mit der gleichen Chlorella-Stammkultur und einer Startkonzentration von 0,2 g/l betrieben.

Die Starttemperatur der Kultivationslösung betrug 26 °C. Während der dreitägigen Kultivation erreichte die Temperatur der Kultivationslösung bis zu 43 °C. Die Farbe der Kultivationslösung hatte sich von grün auf braun-gelb verändert. Eine mikroskopische Untersuchung der Kultivationslösung ergab weniger als 4 % intakte Chlorella-Zellen. Die Biomasse wurde verworfen.

## Patentansprüche

1. Verfahren zur Herstellung von Mikroalgen in einem tubulären Dünnschichtphotobioreaktor, der aus parallel angeordnete Glasröhren besteht, wobei die Mikroalgen ausgehend von einem Misch- und Ausgangsbehälter im Kreislauf geführt werden und dabei in einer Kultivationslösung kultiviert werden, **dadurch gekennzeichnet, dass** die Kultivation der Mikroalgen in dem tubulären Dünnschichtphotobioreaktor bei aktiver Temperaturführung und Kreislaufführung der Kultivationslösung in phototropher oder mixotropher Fahrweise erfolgt.

2. Verfahren nach Anspruch 1, **dadurch gekennzeichnet, dass** zur aktiven Temperaturführung in den Glasröhren ein flexibler und in der Kultivationslösung schwimmender Schlauch verlegt wird, durch den ein Temperiermedium gepumpt wird.

3. Verfahren nach den Ansprüchen 1 und 2, **dadurch gekennzeichnet, dass** das Temperiermedium in dem flexiblen Schlauch periodisch durch Luft ersetzt wird.

4. Verfahren nach den Ansprüchen 1 bis 3, **dadurch gekennzeichnet, dass** durch das Temperiermedium die Kultivationstemperatur oberhalb der minimalen Wachstumstemperatur und unterhalb der maximalen Wachstumstemperatur des jeweilig kultivierten Mikroalgentyps gehalten wird.

5. Verfahren nach einem der Ansprüche 1 bis 4, **dadurch gekennzeichnet, dass** in dem Misch- und Ausgangsbehälter im Kreislauf der Kultivationslösung zur aktiven Temperaturführung ein spiralförmiges Temperierelement zum Kühlen oder Heizen angeordnet wird.

6. Verfahren nach Anspruch 5, **dadurch gekennzeichnet, dass** als spiralförmiges Temperierelement ein aus einem inerten Metallrohr bestehendes Temperierelement eingesetzt wird, welches von einem Temperiermedium durchflossen wird.

7. Verfahren nach einem der Ansprüche 1 und 6, **dadurch gekennzeichnet, dass** der flexible Schlauch und das spiralförmige Temperierelement von dem gleichen Temperiermedium durchflossen werden.

8. Verfahren nach Anspruch 7, **dadurch gekennzeichnet, dass** das Gesamtvolumen des durch den flexiblen Schlauch und das spiralförmige Temperierelement strömenden Temperiermediums zwischen 1 % und 20 %, vorzugsweise 5 % des Kultivationslösungsvolumens beträgt.

9. Verfahren nach Anspruch 1 bis 8, **dadurch gekennzeichnet, dass** durch den flexiblen Schlauch ein anderes Temperiermedium fließt als durch das spiralförmige Temperierelement im Kreislauf der Kultivationslösung.

10. Verfahren nach Anspruch 1 bis 8, **dadurch gekennzeichnet, dass** der flexible Schlauch und/oder das spiralförmige Temperierelement auf ihren äußeren Oberflächen ganz oder teilweise mit einem Schutz gegen die Ablage eines Biofilms versehen werden.

11. Verfahren nach den Ansprüchen 1 bis 10, **dadurch gekennzeichnet, dass** das Verfahren mit dem tubulären Dünnschichtreaktor in einem Gewächshaus durchgeführt wird.

12. Anlage zur Herstellung von Mikroalgen in einem tubulären Dünnschichtphotobioreaktor, wobei die Anlage parallel angeordnete Glasröhren und mindestens einen Misch- und Ausgangsbehälter umfasst, **dadurch gekennzeichnet, dass** die Anlage Mittel zu aktiven Temperaturführung und Kreislaufführung der Kultivationslösung in phototropher oder mixotropher Fahrweise aufweist.

13. Anlage nach Anspruch 12, **dadurch gekennzeichnet, dass** der tubuläre Dünnschichtphotobioreaktor aus Borosilikatglasröhren mit einem Innendurchmesser von 30 bis 70 mm besteht.

14. Anlage nach den Ansprüchen 12 und 13, **dadurch gekennzeichnet, dass** zur aktiven Temperaturführung in den Borosilikatglasröhren ein freiliegender flexibler Schlauchangeordnet ist, der in der Kultivationslösung frei schwimmt und durch den ein Temperiermedium förderbar ist.

15. Anlage nach einem der Ansprüche 12 bis 14, **dadurch gekennzeichnet, dass** in den Misch- und Ausgangsbehälter im Kreislauf der Kultivationslösung zur aktiven Temperaturführung ein spiralförmiges Temperierelement zum Kühlen oder Heizen angeordnet ist.

16. Anlage nach Anspruch 15, **dadurch gekennzeichnet, dass** als spiralförmiges Temperierelement ein aus einem inerten Metallrohr bestehendes Temperierelement angeordnet ist, welches von einem Temperiermedium durchfließbar ist.

17. Anlage nach den Ansprüchen 11 bis 16, **dadurch gekennzeichnet, dass** die äußeren Oberflächen des flexiblen Schlauchs und/oder des spiralförmigen Temperierelementes ganz oder teilweise einen Schutz gegen die Ablage eines Biofilms aufweisen.

18. Anlage nach den Ansprüchen 11 bis 17, **dadurch gekennzeichnet, dass** diese in einem Gewächshaus angeordnet ist.

## Geänderte Patentansprüche

### Geänderte Patentansprüche gemäss Regel 137(2) EPÜ.

1. Verfahren zur Herstellung von Mikroalgen in einem tubulären Dünnschichtphotobioreaktor, der aus parallel angeordnete Glasröhren besteht, wobei die Mikroalgen ausgehend von einem Misch- und Ausgangsbehälter im Kreislauf geführt werden und dabei in einer Kultivationslösung kultiviert werden, wobei die Kultivation der Mikroalgen im tubulären Dünnschichtphotobioreaktor in phototropher oder mixotropher Fahrweise erfolgt, **dadurch gekennzeichnet, dass** die Kultivation bei aktiver Temperaturführung und Kreislaufführung der Kultivationslösung vorgenommen wird, wobei zur aktiven Temperaturführung in den Glasröhren ein flexibler und in der Kultivationslösung schwimmender Schlauch verlegt wird, durch den ein Temperiermedium gepumpt wird und das Temperiermedium in dem flexiblen Schlauch periodisch durch Luft ersetzt wird.

2. Verfahren nach den Anspruch 1, **dadurch gekennzeichnet, dass** durch das Temperiermedium die Kultivationstemperatur oberhalb der minimalen Wachstumstemperatur und unterhalb der maximalen Wachstumstemperatur des jeweilig kultivierten Mikroalgentyps gehalten wird.

3. Verfahren nach den Ansprüchen 1 und 2, **dadurch gekennzeichnet, dass** in dem Misch- und Ausgangsbehälter im Kreislauf der Kultivationslösung zur aktiven Temperaturführung ein spiralförmiges Temperierelement zum Kühlen oder Heizen angeordnet wird.

4. Verfahren nach Anspruch 3, **dadurch gekennzeichnet, dass** als spiralförmiges Temperierelement ein aus einem inerten Metallrohr bestehendes Temperierelement eingesetzt wird, welches von einem Temperiermedium durchflossen wird.

5. Verfahren nach einem der Ansprüche 1 und 3, **dadurch gekennzeichnet, dass** der flexible Schlauch und das spiralförmige Temperierelement von dem gleichen Temperiermedium durchflossen werden.

6. Verfahren nach Anspruch 5, **dadurch gekennzeichnet, dass** das Gesamtvolumen des durch den flexiblen Schlauch und das spiralförmige Temperierelement strömenden Temperiermediums zwischen 1 % und 20 %, vorzugsweise 5 % des Kultivationslösungsvolumens beträgt.

7. Verfahren nach Anspruch 1 bis 6, **dadurch gekennzeichnet, dass** durch den flexiblen Schlauch ein anderes Temperiermedium fließt als durch das spiralförmige Temperierelement im Kreislauf der Kultivationslösung.

8. Verfahren nach Anspruch 1 bis 7, **dadurch gekennzeichnet, dass** der flexible Schlauch und/oder das spiralförmige Temperierelement auf ihren äußeren Oberflächen ganz oder teilweise mit einem Schutz gegen die Ablage eines Biofilms versehen werden.

9. Verfahren nach den Ansprüchen 1 bis 8, **dadurch gekennzeichnet, dass** das Verfahren mit dem tubulären Dünnschichtreaktor in einem Gewächshaus durchgeführt wird.

10. Anlage zur Herstellung von Mikroalgen in einem tubulären Dünnschichtphotobioreaktor, wobei die Anlage parallel angeordnete Glasröhren und mindestens einen Misch- und Ausgangsbehälter umfasst, **dadurch gekennzeichnet, dass** die Anlage Mittel zu aktiven Temperaturführung und Kreislaufführung der Kultivationslösung in phototropher oder mixotropher Fahrweise aufweist, wobei zur aktiven Temperaturführung in den Glasröhren ein freiliegender flexibler Schlauch angeordnet ist, der in der Kultivationslösung frei schwimmt und durch den ein Temperiermedium förderbar ist, wobei das Temperiermedium in dem flexiblen Schlauch periodisch durch Luft ersetzbar ist.

11. Anlage nach Anspruch 10, **dadurch gekennzeichnet, dass** der tubuläre Dünnschichtphotobioreaktor aus Borosilikatglasröhren mit einem Innendurchmesser von 30 bis 70 mm besteht.

12. Anlage nach den Ansprüchen 10 und 11, **dadurch gekennzeichnet, dass** in den Misch- und Ausgangsbehälter im Kreislauf der Kultivationslösung zur aktiven Temperaturführung ein spiralförmiges Temperierelement zum Kühlen oder Heizen angeordnet ist.

13. Anlage nach Anspruch 12, **dadurch gekennzeichnet, dass** als spiralförmiges Temperierelement ein aus einem inerten Metallrohr bestehendes Temperierelement angeordnet ist, welches von einem Temperiermedium durchfließbar ist.

14. Anlage nach den Ansprüchen 10 bis 13, **dadurch gekennzeichnet, dass** die äußeren Oberflächen des flexiblen Schlauchs und/oder des spiralförmigen Temperierelementes ganz oder teilweise einen Schutz gegen die Ablage eines Biofilms aufweisen.

15. Anlage nach den Ansprüchen 10 bis 14, **dadurch gekennzeichnet, dass** diese in einem Gewächshaus angeordnet ist.
